# EUROPÄISCHE PATENTANMELDUNG

(11) **EP 4 321 083 A1**
(43) Veröffentlichungstag der Anmeldung: **14.02.2024**
(21) Anmeldenummer: 23185048.8
(22) Anmeldetag: 12.07.2023
(51) Int. Cl.: A61B 3/00, A61B 3/10, A61B 3/13

(54) **VERFAHREN UND SYSTEM ZUR MULTIMODALEN BILDERFASSUNG UND -VISUALISIERUNG**

(30) Priorität: 10.08.2022 DE 102022120201
(71) Anmelder: Carl Zeiss Meditec AG, 07745 Jena (DE)
(72) Erfinder: Hauger, Christoph, 73431 Aalen (DE); Schmoll, Tilman, 10902 Wien (AT); Hecker-Denschlag, Nancy, 89077 Ulm (DE); Steffen, Joachim, 73463 Westhausen (DE)
(74) Vertreter: Gulde & Partner

(57) **Zusammenfassung**

Die vorliegende Erfindung betrifft ein Verfahren zur multimodalen Bilderfassung und - visualisierung sowie ein System (100) zur multimodalen Bilderfassung und -visualisierung, das System aufweisend ein operationsmikroskopisches System (10) mit einer Optik (11) und einem Bildsensor (12) und ausgebildet zum Erfassen eines zeitaufgelösten Bildsignals (13) eines ausgewählten Sichtfelds (14) einer Probe (15). Das System (100) weist ferner ein OCT-System (20) auf, das zum Erfassen eines zeitaufgelösten OCT-Signals (27) des ausgewählten Sichtfelds (14) ausgebildet ist. Das System weist ein zur zeitaufgelösten Anzeige von Bilddaten (31, 32) ausgebildetes Anzeigemittel (30) und eine Steuereinheit (40) auf, wobei die Steuereinheit (40) dazu eingerichtet ist, zu dem erfassten Bildsignal (13) korrespondierende Videobilddaten (31) zu ermitteln und auf dem Anzeigemittel (30) darzustellen, anhand des erfassten OCT-Signals (27) ein zeitaufgelöstes OCT-Bild (32) zu ermitteln, das zumindest zu einem Abschnitt (60) der dargestellten Videobilddaten (31) korrespondiert und das OCT-Bild (32) an der Position des Abschnitts (60) auf dem Anzeigemittel (30) darzustellen.

## Beschreibung

### Gegenstand der Erfindung

Die vorliegende Erfindung betrifft ein Verfahren und ein System zur multimodalen Bilderfassung, insbesondere zur zeitaufgelösten Bilderfassung eines Operationsgebiets durch die Erfassung von Bildsignalen mittels Video und optischer Kohärenztomographie (*optical coherence tomography*, *OCT*) sowie zur optimierten Darstellung der multimodalen Bilddaten. Bei den multimodalen Bilddaten handelt es sich insbesondere um stereoskopische Bilddaten.

### Technologischer Hintergrund

Der Einsatz von technologischen Hilfsmitteln ist fester Bestandteil der modernen Medizin. Sowohl bildgebende Verfahren als auch robotische Systeme werden mittlerweile in der Chirurgie ebenso selbstverständlich eingesetzt wie in der Diagnostik. Das Verwenden bildgebender Verfahren ermöglicht dabei die Darstellung sowie Diskriminierung vielfältiger Strukturen im Patienten und die vom Patienten gewonnenen Bilddaten können vorteilhaft in der Diagnostik, als auch in therapeutischen und chirurgischen Verfahren eingesetzt werden.

Beispielsweise kann ein Operateur anhand von Bilddaten eines Patienten einen chirurgischen Eingriff besser planen und sich auch bei der Durchführung des Eingriffs unterstützen lassen. Für die Unterstützung von Operateuren bei der Durchführung chirurgischer Eingriffe kommen robotische Visualisierungssysteme zum Einsatz. Diese weisen in der Regel zumindest eine Kamera zur Aufnahme von Bildern des zu operierenden Bereichs auf, die von einem Stativ mit einer Gelenkstruktur getragen wird. Das Stativ erlaubt es, die Kamera durch Translations- und/oder Rotationsbewegungen relativ zum Subjekt zu positionieren, um Bilder eines gewünschten Sichtfelds (*field of view* - *FOV*) des zu operierenden Bereichs zu erfassen. Die Verwendung optischer Stereokameras ermöglicht dabei das Erfassen von 3D Bilddaten.

Neben der Erfassung von Oberflächeninformationen eines gewünschten Sichtfelds, beispielsweise anhand von reflektiertem beziehungsweise rückgestreutem sichtbaren Licht, existieren mittlerweile auch Methoden zum Erfassen von Tiefeninformationen des Sichtfelds. Diese Methoden umfassen die optische Kohärenztomographie (*optical coherence tomography*, *OCT*), welche die dreidimensionale mikroskopische Abbildung von optisch transparenten und/oder reflektierenden Objekten und somit die Aufnahme von Volumenbildern des biologischen Gewebes im betrachteten Sichtfeld erlaubt. Bei der optischen Kohärenztomographie (OCT) handelt es sich im Wesentlichen um eine interferometrische Methode unter Verwendung von breitbandigem Licht mit geringer Kohärenzlänge. Systeme zum Erfassen von OCT-Daten weisen daher ein Interferometer und eine breitbandige Lichtquelle mit einer spektralen Breite von mehr als 1% der zentralen Wellenlänge auf.

Die Erfassung von OCT-Daten kann sequentiell oder parallel erfolgen. Die sequentielle Erfassung von OCT-Daten erfolgt beispielsweise indem ein kohärenzarmer Quelllichtstrahl an einem Strahlteiler in einen Probenstrahl und in einen Referenzstrahl geteilt wird, die durch zwei Arme eines Interferometers geschickt werden, wobei im Referenzstrahlengang ein beweglicher Referenzspiegel und im Objektstrahlengang das zu untersuchende Objekt angeordnet ist. Durch Verschiebung des Referenzspiegels kann ein Gangunterschied zwischen Objekt- und Referenzstrahl und somit die vermessene Probentiefe eingestellt werden. Mittels eines Spiegels im Objektstrahlengang wird der Objektstrahl zweidimensional über die Probe gerastert, was somit eine dreidimensionale Abtastung der Probe ermöglicht.

Bei solch einer Erfassung von OCT-Daten in der Zeitdomäne (*time domain OCT - TD OCT*) korrespondiert die spektrale Breite der Lichtquelle Δλ zu einer Kohärenzlänge Lc von L_{c}=λ*/Δλ. Die axiale Auflösung eines OCT-Systems korrespondiert zur Kohärenzlänge L_{c} des eingesetzten Lichts und bezeichnet das Auflösungsvermögen von Objekten, die entlang der optischen Achse einen Abstand von zumindest der Kohärenzlänge aufweisen. Beispielsweise hat eine Lichtquelle im Nahinfrarotbereich mit einer zentralen Wellenlänge von 800 nm und einer spektralen Breite von 80 nm eine Kohärenzlänge von 7 µm und ein OCT-System mit einer solchen Quelle hat somit eine axiale Auflösung von etwa 1-10 µm. Die transversale Auflösung eines OCT-Systems ist durch die im Objektstrahlengang verwendete Optik bestimmt, insbesondere durch die das Licht auf das zu untersuchende Objekt fokussierende Objektlinse.

Eine sequentielle Erfassung von OCT-Daten ist auch in der Frequenzdomäne möglich (*frequency domain OCT - FD OCT*), wobei in der Regel zwischen der Verwendung einer durchstimmbaren Quelle (*swept source OCT*) und der Verwendung eines dispersiven Detektors (*spectral domain OCT- SD OCT*) unterschieden wird. Bei der *swept source OCT* wird die Frequenz der Anregungslichtquelle, häufig ein Laser, durchgestimmt, wodurch ein Gangunterschied zwischen Proben- und Referenzstrahl und somit die abgetastete Probentiefe auch ohne verschiebbaren Referenzspiegel variiert werden kann. Bei der *SD OCT* wird ebenfalls eine breitbandige Lichtquelle verwendet, jedoch werden die Frequenzkomponenten des Interferenzsignals vor der Detektion separiert, beispielsweise durch ein optisches Gitter.

Mittels OCT sind Schnitt- und Volumendaten von biologischem Gewebe erfassbar und kann der Informationsgehalt für einen Operateur deutlich erhöht werden. Somit ist eine Integration von OCT in Operationsmikroskope wünschenswert, um gleichzeitig Videodaten der Oberfläche eines gewünschten Sichtfelds und Tiefen- und/oder Schnittbilder des Sichtfelds zu erfassen. Die kombinierte Darstellung von intraoperativer OCT-Bildgebung und konventioneller (3D) Bildgebung in Operationsmikroskopen ist jedoch komplex und stellt den Operateur vor neue Herausforderungen. Sofern Operationssysteme bislang dazu in der Lage sind volumetrische OCT-Bilder zu liefern, ist deren Aufnahmezeit relativ hoch und ist das Rendern aus Zeit- und Ressourcengründen zudem allein auf die Nachbearbeitung beschränkt. Eine Volumenbildgebung in Echtzeit während der Operation war daher bislang nicht möglich.

Mit weiteren Fortschritten in der OCT-Technologie sowie der Kapazität und Geschwindigkeit von Prozessoren zur Grafikverarbeitung (GPUs) werden jedoch schnellere OCT-Verfahren zur intraoperativen Bildgebung verfügbar. Die dadurch mögliche Darstellung von OCT-Volumenbildern in Echtzeit kann jedoch nachteilig zur Überforderung beziehungsweise Ablenkung des Operateurs führen, insbesondere wenn OCT und konventionelle Videodaten auf verschiedenen Bildschirmen oder verschiedenen Bildschirmbereichen dargestellt werden beziehungsweise ein Umschalten zwischen den Modalitäten nicht ohne weiteres möglich ist.

Die Aufgabe der vorliegenden Erfindung ist es, die Nachteile des Standes der Technik zu überwinden oder zumindest zu verringern und ein verbessertes Verfahren und ein verbessertes System zur multimodalen Bilderfassung und -visualisierung bereitzustellen.

### Beschreibung der Erfindung

Die erfindungsgemäße Aufgabe wird gelöst durch die Gegenstände der unabhängigen Patentansprüche. Bevorzugte Weiterbildungen sind Gegenstand der Unteransprüche.

Ein erster Aspekt der vorliegenden Offenbarung betrifft ein Verfahren zur multimodalen Bilderfassung und -visualisierung. Dieses weist den Verfahrensschritt des Erfassens eines zeitaufgelösten Bildsignals eines ausgewählten Sichtfelds einer Probe mittels eines operationsmikroskopischen Systems auf. Dabei weist das Bildsignal eine Vielzahl von ersten Tupeln auf, die jeweils ein Oberflächenelement der Probe und zumindest einen zu dem Oberflächenelement korrespondierenden Graustufenwert repräsentieren. Das Verfahren weist ferner den Schritt des Erfassens eines zeitaufgelösten OCT-Signals des ausgewählten Sichtfelds mittels eines OCT Systems auf. Dabei weist das OCT-Signal eine Vielzahl von zweiten Tupeln auf, die jeweils ein Volumenelement der Probe und eine zu dem Volumenelement korrespondierende Streuintensität repräsentieren. Das Verfahren weist ferner den Schritt des Ermittelns von Videobilddaten auf, wobei die Videobilddaten anhand der ersten Tupel und anhand einer Auflösung eines Anzeigemittels mit einer Vielzahl von Pixeln so ermittelt werden, dass in den Videobilddaten bestimmte Pixel bestimmten Oberflächenelementen der Probe zugeordnet werden. In dem Verfahren erfolgt ferner der Schritt des Ermittelns eines zeitaufgelösten OCT-Bilds anhand der zweiten Tupel und anhand der Auflösung des Anzeigemittels und/oder anhand der Videobilddaten. Dabei werden in den OCT-Bildern zu den bestimmten Oberflächenelementen korrespondierende Volumenelemente den bestimmten Pixeln zugeordnet. Somit korrespondiert im Ergebnis das OCT-Bild zumindest zu einem Abschnitt der Videobilddaten. In dem Verfahren erfolgt ferner der Schritt des Darstellens der Videobilddaten auf dem Anzeigemittel und des Darstellens des OCT-Bilds auf dem Anzeigemittel und zwar an der Position des Abschnitts auf dem Anzeigemittel.

Das erfindungsgemäße Verfahren gemäß vorliegender Offenbarung realisiert dieselben Vorteile wie das System gemäß der vorliegenden Offenbarung und diesbezüglich wird auf die dazu untenstehend gemachten Ausführungen verwiesen.

In einer bevorzugten Durchführungsform des Verfahrens gemäß der vorliegenden Offenbarung weist dieses ferner den Schritt des Erfassens eines zeitaufgelösten ersten Bildsignals des Sichtfelds und eines zeitaufgelösten zweiten Bildsignals des Sichtfelds auf. Dieses Erfassen erfolgt bevorzugt mittels einer Stereokamera oder einer anderen zum Erfassen stereoskopischer Bildsignale ausgebildeten Kamera. In der bevorzugten Durchführungsform des Verfahrens erfolgt ferner das Ermitteln zu dem ersten Bildsignal korrespondierender erster Videobilddaten und das Ermitteln zu dem zweiten Bildsignal korrespondierender zweiter Videobilddaten, sprich stereoskopischer Videobilddaten. Ferner erfolgt der Schritt des Ermittelns eines zeitaufgelösten ersten OCT-Bilds, das zumindest zu einem Abschnitt der ersten Videobilddaten korrespondiert, anhand des OCT-Signals, insbesondere anhand der zweiten Tupel und anhand der Auflösung des Anzeigemittels und/oder anhand der Videobilddaten. Ferner erfolgt der Schritt des Ermittelns eines zeitaufgelösten zweiten OCT-Bilds, das zumindest zu dem Abschnitt der zweiten Videobilddaten korrespondiert, anhand des OCT-Signals, insbesondere anhand der zweiten Tupel und anhand der Auflösung des Anzeigemittels und/oder anhand der Videobilddaten. Gemäß dieser Durchführungsform erfolgt ferner das stereoskopisches Darstellen der ersten Videobilddaten, der zweiten Videobilddaten, des zeitaufgelösten ersten OCT-Bilds und des zeitaufgelösten zweiten OCT-Bilds auf dem Anzeigemittel mit gleicher Vergrößerung, gleicher Perspektive und/oder gleichem Stereowinkel. Das Verfahren gemäß dieser Durchführungsform erlaubt vorteilhaft die stereoskopische, gleichzeitige oder sequentielle Darstellung von Videobilddaten und OCT-Bildern an demselben Ort eines Anzeigemittels.

Weitere bevorzugte Durchführungsformen des Verfahrens gemäß der vorliegenden Offenbarung korrespondieren zu weiteren bevorzugten Ausführungsformen des Systems gemäß der vorliegenden Offenbarung und realisieren dieselben Vorteile wie die Ausführungsformen. Es wird auf die dazu untenstehend gemachten Ausführungen verwiesen.

Ein weiterer Aspekt der vorliegenden Offenbarung betrifft ein System zur multimodalen Bilderfassung und -visualisierung, insbesondere zur Bilderfassung und -visualisierung mittels eines medizintechnischen Geräts, beispielsweise eines Operationsmikroskops.

Das System gemäß der vorliegenden Offenbarung weist ein operationsmikroskopisches System auf, welches über eine Optik, insbesondere zum Erfassen von der Probe reflektierten oder rückgestreuten Lichts, beispielsweise sichtbaren Lichts. Die Optik umfasst beispielsweise eine Objektivlinse und ein Okular, kann darüber hinaus jedoch weitere Komponenten umfassen, insbesondere weitere Linsen, Spiegel, Strahlteiler und/oder dergleichen. Das operationsmikroskopische System weist ferner einen Bildsensor auf, der zum Erfassen eines zeitaufgelösten Bildsignals eines ausgewählten Sichtfelds (*region of interest* - *ROI*) einer Probe ausgebildet ist. Bei der Probe handelt es sich beispielsweise um ein Operationsgebiet eines Patienten, bei ophthalmologischen Operationen insbesondere um ein Auge. Bei der Probe kann es sich jedoch auch um beliebige andere Operationsgebiete handeln, wie beispielsweise um Hirngewebe in der Neurochirurgie, um im HNO-Bereich befindliches Gewebe bei der HNO-Chirurgie oder um Zahnfleisch, Zahnstein oder Zahnnerven bei der Dentalchirurgie. Ebenso kann es sich um beliebige andere Gewebe oder Präparate (in vivo, in vitro oder in situ) handeln. Das Sichtfeld wird bevorzugt durch einen Nutzer ausgewählt.

Das System gemäß der vorliegenden Offenbarung weist ferner ein OCT-System auf. Das OCT System umfasst bevorzugt eine breitbandige Lichtquelle, bevorzugt einen durchstimmbaren Laser (*swept source*), beispielsweise einen Breitband-Laser, einen Superkontinuum-Laser und/oder einen Ultrakurzpulslaser. Dabei kann ein durchstimmbarer Laser zu einem gegebenen Zeitpunkt eine schmalbandige Lichtquelle sein, deren Mittelfrequenz jedoch zeitlich gezielt variierbar ist, oder aus einer Mehrzahl schmalbandiger Lichtquellen gebildet sein. Es können jedoch auch andere breitbandige Quellen zum Einsatz kommen, beispielsweise eine Superlumineszenzdiode, beispielsweise in einer *FD-OCT.* Das OCT-System weist ferner bevorzugt ein Interferometer auf, beispielsweise ein Michelson-, Mach-Zehner- oder Koster-Interferometer. Das Interferometer weist bevorzugt einen Strahlteiler zum Erzeugen (und Überlagern) von Proben- und Referenzstrahlen aus dem Licht der breitbandigen Quelle, einen Referenzstrahlengang und einen Probenstrahlengang auf. Ferner bevorzugt weist das Interferometer Mittel zum Einstellen einer untersuchten Probentiefe auf.

Dabei kann es sich je nach Messmethode um ein Mittel zum Erzeugen eines Gangunterschieds (wie eines im Referenzstrahl verschiebbaren Spiegel bei einer SD-OCT), ein Mittel zum Separieren von Licht eines bestimmten Gangunterschieds (wie ein optisches Gitter bei einer FD-OCT) oder um Mittel zum Erzeugen von Licht eines bestimmten Gangunterschieds (wie eine durchstimmbaren Quelle bei einer *swept source-OCT*) handeln.

Das OCT-System weist ferner bevorzugt einen Scan-Mechanismus für den Probenstrahl auf, insbesondere um den Probenstrahl in zwei Dimensionen über die Probe zu rastern. Bevorzugt handelt es sich bei dem Scanmechanismus um einen Scanspiegel, es können jedoch auch andere Scanmechanismen, wie beispielsweise ein Gasfaserscanner, ein Prismenscanner, ein Palmer-Scanner oder dergleichen zum Einsatz kommen. Bei einem für Full-Field-OCT eingerichteten OCT-System ist ein Scanmechanismus verzichtbar. Das OCT-System ist zum Erfassen eines zeitaufgelösten OCT-Signals des ausgewählten Sichtfelds ausgebildet und weist hierfür bevorzugt einen Detektor auf. Bei dem Detektor handelt es sich beispielsweise um einen Liniendetektor, ein zweidimensionales Detektorarray, einen Photodetektor, einen dispersiven Detektor, einen CCD-Detektor und/oder einen CMOS-Detektor.

Das System gemäß der vorliegenden Offenbarung weist ferner ein zur zeitaufgelösten Anzeige von Bilddaten ausgebildetes Anzeigemittel auf. Bei dem Anzeigemittel handelt es sich bevorzugt um einen oder mehrere Bildschirme, beispielsweise um den zumindest einen Bildschirm eines Operationsmikroskops, um einen in einem Operationsraum fest installierten Bildschirm oder um ein Head-Mounted-Display (HMD), beispielsweise eine Videobrille. Bei dem Bildschirm handelt es sich vorzugsweise um einen 4K und/oder 8K fähigen Bildschirm und/oder um einen zur stereoskopischen Darstellung ausgebildeten 3D-Bildschirm.

Das System gemäß der vorliegenden Offenbarung weist ferner eine Steuereinheit auf, die mit dem operationsmikroskopischen System, dem OCT-System und dem Anzeigemittel verbunden ist, insbesondere zur ein- oder wechselseitigen Datenübertragung. Die Steuereinheit ist dazu ausgebildet und eingerichtet, zu dem erfassten zeitaufgelösten Bildsignal korrespondierende Videobilddaten zu ermitteln. Bei dem zeitaufgelösten Bildsignal handelt es sich insbesondere um eine Vielzahl von Oberflächenelementen der Probe zugeordneten Signalen, die sequentiell oder simultan für ein bestimmtes Raster der Probenoberfläche erfasst sind, wobei das Raster durch einen Scanmechanismus und/oder den Bildsensor bestimmt ist. Das zeitaufgelöste Bildsignal weist ferner eine Taktfrequenz (Bildwiederholrate) auf, die durch den Scanmechanismus und/oder den Bildsensor bestimmt ist. Die Steuereinheit erzeugt aus diesem Bildsignal Videobilddaten mit einer einem Raster (Auflösung) und einer Bildwiederholrate die zur Darstellung auf dem Anzeigemittel geeignet ist. Die Steuereinheit ist ferner dazu eingerichtet die Videobilddaten mittels des Anzeigemittels darzustellen.

Die Steuereinheit ist ferner dazu eingerichtet, anhand des OCT-Signals ein zeitaufgelöstes OCT-Bild zu ermitteln. Bei dem OCT-Signal handelt es sich um ein Interferenzsignal, wobei die Modulation der Einhüllenden des Interferenzsignals Reflektionseigenschaften der Probe kodiert. Mittels des Scanmechanismus kann die Probe zweidimensional in einer über den Gangunterschied eingestellten Probentiefe abgerastert werden. Anhand des eingesetzten Scanmechanismus, dem eingesetzten Mittel zum Auswählen beziehungsweise Erzeugen des Gangunterschieds, beispielsweise eines verstellbaren Spiegels im Referenzstrahl, einem optischen Gitter vor dem Detektor oder einer durchstimmbaren breitbandigen Lichtquelle und der Wiederholratze des Detektors ergibt sich auch für das zeitaufgelöste OCT-Signal eine Taktfrequenz (Bildwiederholrate). Die Steuereinheit ermittelt anhand des OCT-Signals rechnerisch ein zeitaufgelöstes OCT-Bild, beispielsweise mittels Volumen-Rendering, Ray-Tracing und/oder Ray-Marching. Dem Fachmann sind diverse Verfahren zum Erzeugen von zeitaufgelösten OCT-Bildern aus zeitaufgelösten OCT-Signalen bekannt.

In dem System gemäß der vorliegenden Offenbarung weist ein Bildsignal eine Vielzahl von ersten Tupeln auf. Dabei umfasst (beziehungsweise repräsentiert) jedes erste Tupel ein Oberflächenelement der Probe und zumindest einen Graustufenwert. Das Oberflächenelement der Probe ist dabei bevorzugt durch zwei laterale Raumkoordinaten (beispielsweise x und y) repräsentiert und kann beispielsweise als ein Probenpixel interpretiert werden. Neben dem Graustufenwert, der sich letztlich aus einer erfassten Intensität ergibt, kann jedes erste Tupel darüber hinaus auch Farbwerte aufweisen, beispielsweise bei der Erfassung von Intensitäten für verschiedene Farben mittels dem Bildsensor vorgeschalteten Farbfiltern. Ferner weist ein OCT-Signal eine Vielzahl von zweiten Tupeln auf, die jeweils ein Volumenelement der Probe und eine Streuintensität umfassen (beziehungsweise repräsentieren). Das Volumenelement der Probe ist dabei bevorzugt durch drei Raumkoordinaten (beispielsweise *x*, *y* und *z*) repräsentiert und kann beispielsweise als ein Probenvoxel interpretiert werden. Neben der Streuintensität kann das zweite Tupel weitere Werte aufweisen. Gemäß der vorliegenden Offenbarung weist das Anzeigemittel eine Vielzahl von Pixeln auf und ist die Steuereinheit dazu eingerichtet, die Videobilddaten anhand der ersten Tupel und anhand einer Auflösung des Anzeigemittels so zu ermitteln, dass bestimmte Pixel bestimmte Oberflächenelemente der Probe anzeigen. Mit anderen Worten ermittelt die Steuereinheit eine erste Zuordnung von Pixeln des Anzeigemittels und Oberflächenelementen der Probe. Diese Zuordnung kann dabei von weiteren Einstellungen abhängen, wie beispielsweise einer Zoomstufe des operationsmikroskopischen Systems, ist aber für gegebene Einstellungen bevorzugt zeitlich konstant. Die Steuereinheit der vorliegenden Offenbarung ist ferner dazu eingerichtet, die OCT-Bilder anhand der zweiten Tupel und zumindest einem der Auflösung des Anzeigemittels und/oder der Videobilddaten so zu ermitteln, dass zu den bestimmten Oberflächenelementen korrespondierende Volumenelemente auf den bestimmten Pixeln dargestellt werden. Mit anderen Worten ermittelt die Steuereinheit eine zweite Zuordnung von Pixeln des Anzeigemittels und Volumenelementen der Probe, welche zu der ermittelten ersten Zuordnung korrespondiert. Die Steuereinheit realisiert somit eine örtliche Registrierung zwischen Pixeln des Anzeigemittels und den Bildsignalen (Videobilddaten) als auch den OCT-Signalen (OCT-Bildern).

Im System gemäß der vorliegenden Offenbarung erzeugt die Steuereinheit das OCT-Bild derart, dass das erzeugte zeitaufgelöste OCT-Bild zumindest zu einem Abschnitt der dargestellten Videobilddaten korrespondiert. Bevorzugt wird ein OCT-Signal von dem gesamten Sichtfeld erfasst und wird ein OCT-Bild von zumindest einem Teil des Sichtfelds erstellt. Ebenso bevorzugt wird ein OCT-Signal von einem Abschnitt des Sichtfelds erfasst und wird ein OCT-Bild von zumindest einem Teil des Abschnitts des Sichtfelds erstellt. Die örtliche Korrespondenz von Videobilddaten und OCT-Bild wird erfindungsgemäß entweder anhand der eingesetzten Messtechnik oder durch bildverarbeitende Methoden gewährleistet. Die Steuereinheit ist ferner dazu ausgebildet, das zeitaufgelöste OCT-Bild an der Position des Abschnitts der Videobilddaten auf dem Anzeigemittel darzustellen. Bevorzugt werden Videobilddaten und ein OCT-Bild von dem gesamten Sichtfeld erstellt und jeweils auf dem gesamten Anzeigemittel dargestellt. Ebenso bevorzugt wird ein OCT-Bild von einem Abschnitt des Sichtfelds erzeugt und an der Position der zu diesem Abschnitt des Sichtfelds korrespondierenden Videodaten auf dem Anzeigemittel dargestellt. Mit anderen Worten werden Videobilddaten und OCT-Bilder, die zu demselben Abschnitt der Probe korrespondieren an derselben Stelle des Anzeigemittel dargestellt. Das System gemäß der vorliegenden Offenbarung ermöglicht somit eine nahtlose Integration von Videobilddaten und OCT-Bildern auf dem Anzeigemittel, wodurch einem Nutzer eine einfachere Betrachtung der multimodalen Bilddaten ermöglicht wird. So können die multimodalen Bilddaten betrachtet werden, ohne den Kopf oder die Augen zu bewegen, was sich insbesondere bei der Bildgebung während operativer Eingriffe vorteilhaft auf die Aufmerksamkeit des Operateurs auswirkt.

In einer besonders bevorzugten Ausführungsform des Systems gemäß der vorliegenden Offenbarung weist das operationsmikroskopische System eine Stereokamera auf, ist also zum Erfassen von stereoskopischen zeitaufgelösten Bildsignalen ausgebildet. Hierzu weist die Stereokamera insbesondere einen ersten Bildsensor zum Erfassen eines zeitaufgelösten ersten Bildsignals des Sichtfelds und einen zweiten Bildsensor zum Erfassen eines zeitaufgelösten zweiten Bildsignals des Sichtfelds auf. Der erste Bildsensor erfasst das erste Bildsignal bevorzugt entlang einer ersten optischen Achse und mittels einer ersten Optik, insbesondere einem ersten Objektiv. Der zweite Bildsensor erfasst das zweite Bildsignal bevorzugt entlang einer zweiten optischen Achse und mittels einer zweiten Optik, insbesondere einem zweiten Objektiv. Die erste optische Achse und die zweite optische Achse schließen dabei einen Winkel miteinander ein. Dieser Winkel zwischen den optischen Achsen sowie ein Arbeitsabstand zwischen der Probe und den Objektiven bestimmen dabei einen Stereowinkel der stereoskopischen Bilderfassung. Gemäß dieser Ausführungsform ist das Anzeigemittel zum Darstellen stereoskopischer Bilddaten ausgebildet, wobei der Stereowinkel maßgeblich für den Tiefeneindruck bei der Bildvisualisierung auf dem Anzeigemittel ist.

Ferner bevorzugt ist die Steuereinheit des erfindungsgemäßen Systems dazu eingerichtet, zu dem ersten Bildsignal korrespondierende erste Videobilddaten und zu dem zweiten Bildsignal korrespondierende zweite Videobilddaten zu erzeugen, jeweils wie obenstehend beschrieben, und diese auf dem Anzeigemittel stereoskopisch darzustellen. Das Anzeigemittel ist somit dafür ausgebildet, Bilddaten stereoskopisch darzustellen. Bei dem Anzeigemittel handelt es sich bevorzugt um ein Anzeigemittel, das zur Anzeige von Bilddaten mit verschiedenen (beispielsweise orthogonalen) Polarisationen ausgebildet ist, in Kombination mit einer Polarisationsbrille. Bei dem Anzeigemittel kann es sich jedoch auch um einen 3D-Bildschirm, beispielsweise einen Lichtfeldmonitor oder dergleichen, handeln. Die Steuereinheit ist ferner dazu eingerichtet, anhand des erfassten zeitaufgelösten OCT-Signals ein zeitaufgelöstes erstes OCT-Bild und ein zeitaufgelöstes zweites OCT-Bild zu ermitteln. Das OCT-System bietet mithin die Möglichkeit zum Erzeugen stereoskopischer Bilddaten ohne die Notwendigkeit einer stereoskopischen Signalerfassung. Gemäß dieser bevorzugten Ausführungsform korrespondiert das erste OCT-Bild zumindest zu einem Abschnitt der dargestellten ersten Videobilddaten und korrespondiert das zweite OCT-Bild zumindest zu dem gleichen Abschnitt der dargestellten zweiten Videobilddaten, jeweils wie obenstehend beschrieben. Die Steuereinheit ist ferner dazu ausgebildet, das erste und das zweite OCT-Bild an der Position des Abschnitts auf dem Anzeigemittel stereoskopisch darzustellen. Somit ergeben sich die erfindungsgemäßen Vorteile einer optimierten multimodalen Bildvisualisierung auch im Rahmen einer stereoskopischen Darstellung von Bilddaten. Bevorzugt berücksichtigt die Steuereinheit bei der Erzeugung der stereoskopischen OCT-Bilder den Stereowinkel des operationsmikroskopischen Systems. Somit wird vorteilhaft ein identischer Tiefeneindruck der stereoskopischen Videobilddaten und der stereoskopischen OCT-Bilder gewährleistet.

Ferner bevorzugt ist die Steuereinheit dazu eingerichtet, die durch das operationsmikroskopische System erfassten Bildsignale und die durch das OCT-System erfassten OCT-Signale anhand von Erfassungsparametern des OCT-Systems und des operationsmikroskopischen Systems örtlich zu registrieren. Eine örtliche Registrierung dieser Signale bezeichnet dabei eine korrekte Verknüpfung dieser Signale mit einem Referenzkoordinatensystem, beispielsweise dem Koordinatensystem des Patienten während eines Eingriffs und ermöglicht eine eindeutige Abbildung (Mapping) von Koordinaten des Patientenraumes auf entsprechende Koordinaten des Signalraumes. Eine Registrierung der Signale erfordert bevorzugt eine Kalibrierung von dem operationsmikroskopischen System und OCT System. Die Erfassungsparameter des operationsmikroskopischen Systems umfassen bevorzugt Kalibrierungsparameter und/oder optische Einstellungen des operationsmikroskopischen Systems, wie beispielsweise eine Fokuslänge und/oder eine Zoomstufe einer eingesetzten Optik (Kamera). Darüber hinaus umfassen die Erfassungsparameter bevorzugt auch einen Satz intrinsischer Parameter des operationsmikroskopischen Systems. Die intrinsischen Parameter bestimmen dabei einen Zusammenhang zwischen dem Koordinatensystem eines Bildsignals und dem Koordinatensystem des zugehörigen bildgebenden Sensors. Die Art der intrinsischen Parameter hängt dabei insbesondere von der Art des eingesetzten bildgebenden Sensors ab, wobei mit bildgebendem Sensor hier sowohl der eigentliche Sensor als auch die verwendete Optik bezeichnet ist. Die intrinsischen Parameter umfassen, beispielsweise bei einer dem Fachmann bekannten Kamerakalibrierung nach Tsai, eine effektive Brennweite, die Koordinaten eines Bildhauptpunktes (Zentrum der Verzerrung) eines Bildsignals, einen ersten Skalierungsfaktor und/oder einen ersten radialen Linsenfehlerkoeffizienten (Verzerrungskoeffizient). Alternativ zu den vorgenannten intrinsischen Parametern einer Kamerakalibrierung nach Tsai können auch andere intrinsische Parameter, beispielsweise für eine Kamerakalibrierung nach Zhang verwendet werden (vgl. beispielsweise "A practical comparison between Zhang's and Tsai's calibration approaches", Li et al., Proceedings of the 29th International Conference on Image and Vision Computing New Zealand, November 2014 Pages 166-171, DOI:10.1145/2683405.2683443). Das OCT-System weist ebenfalls Erfassungsparametern auf, die bevorzugt Kalibrierungsparameter umfassen. Die Erfassungsparameter des OCT-Systems berücksichtigen bevorzugt auch den Scanmechanismus und/oder den Detektor des OCT-Systems. Anhand der örtlichen Registrierung anhand der Erfassungsparameter können vorteilhaft an definierten Koordinaten des Patientenraums befindliche Strukturen des Patienten an den entsprechenden Koordinaten im Bildraum der Videobilddaten und im Bildraum der OCT-Bilder korrekt dargestellt werden.

In einer ebenfalls bevorzugten Ausführungsform des Systems gemäß der vorliegenden Offenbarung ist die Steuereinheit alternativ oder zusätzlich dazu ausgebildet, die Videobilddaten und OCT-Bilder mittels Bildanalyse örtlich zu registrieren. Dabei bezeichnet eine örtliche Registrierung dieser Bilddaten eine korrekte Verknüpfung dieser Bilder in einem gemeinsamen Bildkoordinatensystem. Die Registrierung anhand der Bilddaten ermöglicht somit eine relative Verknüpfung der Bilddaten für eine möglichst deckungsgleiche Abbildung gleicher Strukturen. Beispielsweise können Struktur- oder Gewebegrenzen in den Videobilddaten und den OCT-Bildern mittels Bildanalyse, beispielsweise Kantenerkennung oder dergleichen, erkannt und miteinander verglichen werden. Durch translatorische Verschiebung, Rotation und/oder Skalierung können diese Strukturen dann auf dem Anzeigemittel miteinander in Überlagerung gebracht werden. Bevorzugt erfolgt die örtliche Registrierung der Bilddaten zusätzlich zu einer örtlichen Registrierung der erfassten Signale.

Die Steuereinheit des Systems ist bevorzugt dazu eingerichtet, die Videobilddaten und das zeitaufgelöste OCT-Bild gleichzeitig auf dem Anzeigemittel darzustellen. Dies ermöglicht besonders vorteilhaft die gleichzeitige Berücksichtigung beider Bildmodalitäten durch einen Anwender. Um dennoch eine Unterscheidung der verschiedenen Bilddaten zu ermöglichen, ist die Steuereinheit ferner bevorzugt dazu eingerichtet, die Videobilddaten mit einer ersten Transparenzstufe und das zeitaufgelöste OCT-Bild mit einer zweiten Transparenzstufe darzustellen. Dabei unterscheiden sich die erste und die zweite Transparenzstufe bevorzugt.

Ebenfalls bevorzugt variieren die erste und zweite Transparenzstufe zeitlich. Beispielsweise wird zunächst das Bildsignal mit einer Transparenz von 0% dargestellt, während das OCT-Bild mit einer Transparenz von 100% dargestellt wird. Im zeitlichen Verlauf wird die Transparenz des Bildsignals dann kontinuierlich von 0% auf 100% eingestellt, während zeitgleich die Transparenz des OCT-Bildes von 100% auf 0% eingestellt wird. Somit wird ein kontinuierlicher Übergang zwischen der Darstellung des Videobildes und der des OCT-Bildes gewährleistet.

In einer ebenfalls bevorzugten Ausführungsform des Systems gemäß der vorliegenden Offenbarung ist die Steuereinheit dazu eingerichtet, die Videobilddaten und das zeitaufgelöste OCT-Bild sequentiell auf dem Anzeigemittel darzustellen. Mit anderen Worten wird, zumindest an einem bestimmten Ort des Anzeigemittels, zu jeder Zeit nur eines von Videobilddaten und OCT-Bildern dargestellt. Dies ermöglicht vorteilhaft eine deutliche Unterscheidung zwischen Videobilddaten und OCT-Daten und ebenso vorteilhaft eine gleichzeitige Darstellung der verschiedenen Bilddaten an verschiedenen Orten des Anzeigemittels.

Besonders bevorzugt ist die Steuereinheit dazu eingerichtet, die Videobilddaten und das zeitaufgelöste OCT-Bild mit gleicher Vergrößerung, gleicher Perspektive und/oder gleichem Stereowinkel darzustellen. Dies ermöglicht bei einer gleichzeitigen Darstellung beider Videobilddaten am selben Ort des Anzeigemittels bevorzugt eine perfekte Überlagerung beider Bilddaten und kann vorteilhaft eine Darstellung mit optimiertem Kontrast ermöglichen. Bei einer sequentiellen Darstellung erfolgt bevorzugt am Übergang zwischen den örtlich registrierten Bilddaten eine Darstellung mit gleicher Vergrößerung, gleicher Perspektive und/oder gleichem Stereowinkel. Somit erfolgt ein flüssiger Übergang zwischen der Darstellung der Bilddaten. Bei den Videobilddaten ist lediglich eine Darstellung der Oberfläche (Draufsicht) möglich. Dies entspricht beispielsweise einem "*en-face*" OCT-Bild. Sobald ein Übergang von Videobilddaten zu OCT-Bild erfolgt ist, kann ein Nutzer ferner bevorzugt Vergrößerung, Perspektive und/oder Stereowinkel anpassen, um vorteilhaft eine verbesserte Ansicht mit optimaler Tiefenwahrnehmung zu ermöglichen. Insbesondere kann ein Nutzer von der initialen "en-face"Ansicht auf eine perspektivische Darstellung, beispielsweise durch einen kontinuierlichen Übergang der Blickrichtung oder auf eine Schnittansicht (OCT-B-Scan) wechseln. Somit wird in verschiedenen Phasen vorteilhaft die perfekte Ansicht gewährleistet.

In dem System gemäß der vorliegenden Offenbarung korrespondiert das zeitaufgelöste OCT-Bild bevorzugt zu einem Abschnitt der dargestellten Videobilddaten. Mit anderen Worten stellt das OCT-Bild lediglich einen Abschnitt des mittels des operationsmikroskopischen Systems betrachteten Sichtfelds dar. Beispielsweise kann ein zeitaufgelöstes OCT-Signal nur von einem Abschnitt des betrachteten Sichtfelds der Probe erfasst werden. Gemäß dieser bevorzugten Ausführungsform ist die Steuereinheit dazu eingerichtet, das OCT-Bild in dem Abschnitt und die Videobilddaten auf dem Rest des Anzeigemittels darzustellen. Beispielsweise werden die Videobilddaten und das OCT-Bild auf dem Anzeigemittel insgesamt gleichzeitig auf den spezifischen Orten des Anzeigemittels aber sequentiell angezeigt. Dies ermöglicht vorteilhaft eine Anzeige über die gesamte Probe beziehungsweise einen gesamten Operationsbereich mittels der Videobilddaten, während gleichzeitig ein Ausschnitt beziehungsweise ein Detail, beispielsweise der Eingriffsbereich eines chirurgischen Instruments, mittels der OCT-Bilder dargestellt wird. Da die OCT-Bilder auch eine perspektivische Darstellung ermöglichen, ermöglicht diese Ausführungsform vorteilhaft auch die Kombination einer Draufsicht in den Videobilddaten mit der perspektivischen OCT-Sicht.

In einer besonders bevorzugten Ausführungsform weist das System gemäß der vorliegenden Offenbarung ferner eine Schnittstelle zum Erfassen einer Nutzereingabe auf. Bei der Schnittstelle handelt es sich bevorzugt um einen Handschalter oder um einen Fußschalter. Ebenso bevorzugt handelt es sich bei der Schnittstelle um ein Mittel zum Erkennen einer Kopf- und/oder Augenbewegung, beispielsweise integriert in eine Videobrille oder Head-Mounted-Display, HMD. Die Schnittstelle kann ferner zum Erfassen von Sprachbefehlen ausgebildet sein und hierfür zumindest ein Mikrofon umfassen. Ebenso bevorzugt handelt es sich bei der Schnittstelle um eine Tastatur, einen Joystick, eine Maus, einen Touchscreen oder eine Kombination davon. Gemäß dieser Ausführungsform ist die Steuereinheit dazu eingerichtet, einen Wechsel der Darstellung von Videobilddaten zu OCT-Bild beziehungsweise von OCT-Bild zu Videobilddaten anhand einer erfassten Nutzereingabe zu initiieren.

In einer ebenfalls besonders bevorzugten Ausführungsform des Systems gemäß der vorliegenden Offenbarung weist dieses ferner ein medizintechnisches Instrument auf. Bei dem medizintechnischen Instrument handelt es sich beispielsweise um eine Sonde, einen Pointer, eine Pinzette, eine Ahle, einen Phako-Tip, ein Endoskop, eine Endo-LED oder dergleichen.

Gemäß dieser Ausführungsform ist die Steuereinheit ferner dazu eingerichtet, eine Position, einen Typ und/oder einen Zustand des medizintechnischen Instruments zu ermitteln. Ein Typ des medizintechnischen Instruments kann dabei bevorzugt anhand einer Schnittstelle zum Anschluss des medizintechnischen Instruments und/oder anhand einer Eingabe durch eine Nutzerschnittstelle erfolgen. Ebenso bevorzugt erfolgt die Erkennung eines Typs des in das Sichtfeld des operationsmikroskopischen Systems eingebrachten medizintechnischen Instruments durch Bildanalyse der Videobilddaten, beispielsweise mittels Segmentierung und Objekterkennung. Eine Position des medizintechnischen Instruments wird bevorzugt anhand der Detektion eines Markers ermittelt, wobei der Marker eine Kennzeichnung auf oder eine Struktur des medizintechnischen Instruments sein kann. Die Erfassung des Markers erfolgt bevorzugt mittels des operationsmikroskopischen Systems und gegebenenfalls unter Verwendung zusätzlicher Lichtquellen (beispielsweise Infrarot-LEDs) und/oder nach einer Registrierung/Kalibrierung des medizintechnischen Instruments (beispielsweise durch Positionieren einer Spitze des medizintechnischen Instruments an einem definierten Ort).

Ein Zustand des in das Sichtfeld des operationsmikroskopischen Systems eingebrachten medizintechnischen Instruments wird bevorzugt ebenfalls anhand von Bildanalyse der Videobilddaten ermittelt. So ist beispielsweise anhand der Bilddaten zu erkennen, ob eine Pinzette geöffnet oder geschlossen ist. Darüber hinaus kann eine Nutzereingabe zum Ändern eines Zustands von der Steuereinheit eingelesen werden, beispielsweise signalisiert eine Nutzereingabe zum Aktivieren eines Phako-Tips eine Änderung von dessen Zustand. Ferner kann ein an dem medizintechnischen Instrument angebrachter Sensor die Änderung von dessen Zustand detektieren, beispielsweise das Schließen einer Pinzette und ein entsprechendes Sensorsignal an die Steuereinheit übermitteln.

Gemäß dieser bevorzugten Ausführungsform ist die Steuereinheit ferner dazu eingerichtet, einen Wechsel der Darstellung von Videobilddaten zu zeitaufgelöstem OCT-Bild beziehungsweise von zeitaufgelöstem OCT-Bild zu Videobilddaten anhand der Position, des Typs und/oder des Zustands des medizintechnischen Instruments zu initiieren. Ein Wechsel des Zustands eines medizintechnischen Instruments eines bestimmten Typs und/oder an einem bestimmten Ort ist bevorzugt indikativ für eine bestimmte Phase einer Operation. Mithin kann anhand der Erkennung von Position, des Typs und/oder des Zustands des medizintechnischen Instruments die für diese Phase optimale Darstellungsform gewählt werden. Dies umfasst neben dem Wechsel der Bilddaten gegebenenfalls auch einen Wechsel von Darstellungsparametern innerhalb der Bilddaten, beispielsweise einer Zoomstufe, eines Stereowinkels, einer Blickrichtung, einer dargestellten Tiefe und/oder einer Schnittrichtung.

Ebenso bevorzugt ist die Steuereinheit dazu eingerichtet, eine Zoomstufe des operationsmikroskopischen Systems zu ermitteln und einen Wechsel der Darstellung von Videobilddaten und von zeitaufgelöstem OCT-Bild anhand der Zoomstufe zu initiieren. Ein kleiner Zoomwert korrespondiert in der Regel zu einem großen Sehfeld, wobei eine Darstellung von Details in der Regel nicht gewünscht ist. Daher wird anhand einer kleinen Zoomstufe bevorzugt darauf geschlossen, dass eine Darstellung von OCT-Bildern nicht gewünscht ist. Ein großer Zoomwert korrespondiert hingegen zu einem kleinen Sehfeld, in dem häufig eine Detaildarstellung gewünscht ist. Daher wird anhand einer kleinen Zoomstufe bevorzugt darauf geschlossen, dass eine Darstellung von OCT-Bildern gewünscht ist.

Ferner bevorzugt ist die Steuereinheit dazu eingerichtet, eine Phase einer durchgeführten Operation zu ermitteln. Hierzu ist die Steuereinheit bevorzugt mit einem Speicher verbunden, in dem ein trainierter Algorithmus des maschinellen Lernens, beispielsweise ein neurales Netzwerk (CNN) oder dergleichen, abgelegt ist. Dieser Algorithmus wurde bevorzugt mittels einer Vielzahl von Videobilddaten und/oder OCT-Bildern beziehungsweise Bildsignalen und/oder OCT-Signalen trainiert, denen im Training eine korrespondierende Phase einer OP als Klassifikation zugeordnet worden ist. Demzufolge ist der trainierte Algorithmus in der Lage, anhand von Videobilddaten und/oder OCT-Bildern beziehungsweise Bildsignalen und/oder OCT-Signalen selbständig eine Phase einer Operation als Klassifikation zu erkennen. Bevorzugt ist die Steuereinheit gemäß dieser Ausführungsform dazu ausgebildet, einen Wechsel der Darstellung von Videobilddaten zu OCT-Bild beziehungsweise von OCT-Bild zu Videobilddaten anhand der ermittelten Phase der Operation zu initiieren. Die für verschiedene Phasen der Operation geeigneten Bilddaten sind bevorzugt in einem Speicher hinterlegt.

Bei einer Kataraktoperation können die Operationsphasen beispielsweise umfassen: Ruhezustand, Inzision, Injektion des ophthalmischen viskochirurgischen Geräts (OVD), Kapsulorhexis, Hydrodissektion, Phakoemulsifikation, Spülung/Aspiration, Implantation der Intraokularlinse, Schließen/Befeuchten der Wunde, Nichtoperation. Bei einer refraktiven Operation können die Operationsphasen beispielsweise umfassen: Leerlauf, Andocken, Applanation, Anlegen des Auges/CG-Drehung, Linsenschnitt, Linsenseitenschnitt, Kappenschnitt, Kappenseitenschnitt, Freigeben des Auges, Übergang zum OPMI, Positionierung des OPMI, Eröffnung der Inzision, Festlegung der Ebenen, Abtrennung des Kappenbetts, Abtrennung des Linsenbetts, Entfernung und/oder Inspektion der Linsen, Abwischen, Spülen, Spaltlampe, Entfernung des Spekulums. Bei einem zahnärztlichen Eingriff können die chirurgischen Phasen beispielsweise umfassen: Zugang, Exstirpation, Debridement, Trocknung, Obturation, Restauration. Es ist zu beachten, dass alle oder nur einige dieser Phasen Teil der entsprechenden Operation sein können und dass auch weitere Operationsphasen vorhanden sein und/oder einige Phasen ausgelassen werden können.

Die Funktionalitäten der erfindungsgemäßen Steuereinheit können durch elektrische oder elektronische Bauteile oder Komponenten (Hardware), durch Firmware (ASIC) implementiert sein und/oder durch Ausführen eines geeigneten Programms (Software) verwirklicht werden. Bevorzugt werden die Funktionalitäten der erfindungsgemäßen Steuereinheit durch eine Kombination von Hardware, Firmware und/oder Software verwirklicht, beziehungsweise implementiert. Beispielsweise sind einzelne Komponenten der erfindungsgemäßen Steuereinheit zum Ausführen einzelner Funktionalitäten als separat integrierter Schaltkreis ausgebildet oder auf einem gemeinsamen integrierten Schaltkreis angeordnet.

Die einzelnen Funktionalitäten der erfindungsgemäßen Steuereinheit sind ferner bevorzugt als ein oder mehrere Prozesse ausgebildet, die auf einem oder mehreren Prozessoren in einem oder mehreren elektronischen Rechengeräten laufen und beim Ausführen von ein oder mehreren Computerprogrammen erzeugt werden. Die Steuereinheit ist dabei dazu ausgebildet, mit den anderen Komponenten, insbesondere dem operationsmikroskopischen System, dem OCT-System und dem Anzeigemittel zusammenzuarbeiten, um die hierin beschriebenen Funktionalitäten des erfindungsgemäßen Systems zu verwirklichen. Dem Fachmann ist ferner ersichtlich, dass die Funktionalitäten von mehreren Computern (Datenverarbeitungsgeräten, Steuereinheiten, Steuergeräte) kombiniert oder in einem einzigen Gerät kombiniert sein können oder dass die Funktionalität von einem bestimmten Datenverarbeitungsgerät auf eine Vielzahl von Geräten verteilt vorliegen könne, um die Funktionalitäten der erfindungsgemäßen Steuereinheit zu realisieren.

In einer besonders bevorzugten Ausführungsform des erfindungsgemäßen Systems ist dieses in ein Operationsmikroskop integriert. Dabei weist das Operationsmikroskop bevorzugt ein operationsmikroskopisches System und ein OCT-System, jeweils wie obenstehend beschrieben, auf. Ferner bevorzugt weist das Operationsmikroskop ein Anzeigemittel auf oder ist es mit einem solchen verbunden. Bevorzugt sind Bildsensor und Optik Teil einer Kamera, insbesondere einer Hauptbeobachterkamera oder einer Umfeldkamera des Operationsmikroskops. Die Steuereinheit des Operationsmikroskops ist bevorzugt als Steuereinheit des erfindungsgemäßen Systems ausgebildet und ist insbesondere dazu ausgebildet, anhand von auf einer Speichereinheit des Operationsmikroskops gespeicherten Befehlen das erfindungsgemäße Verfahren wie nachfolgend beschrieben durchzuführen.

Unter einem Operationsmikroskop wird im Rahmen der vorliegenden Offenbarung im weitesten Sinne ein für den Einsatz während einer Operation geeignetes Mikroskop verstanden. Das Operationsmikroskop weist bevorzug eine Halterung auf, die eine Abbildung des Operationsgebiets unabhängig von Kopfbewegungen des Operateurs erlaubt. Ferner bevorzugt weist das Operationsmikroskop zumindest einen Strahlteiler und zumindest zwei Okulare auf. Alternativ handelt es sich bei dem Operationsmikroskop um ein reines "Digiskop" ohne Okulare. Ebenfalls bevorzugt weist das Operationsmikroskop zumindest einen bildgebenden Sensor auf. Ferner bevorzugt weist das Operationsmikroskop eine Hauptbeobachterkamera und eine Umfeldkamera auf. Das Operationsmikroskop kann kinematische beziehungsweise robotische Hilfsmittel zum Durchführen operativer Eingriffe aufweisen. Alternativ kann ein Operationsmikroskop als medizintechnisches Mikroskop, medizinisch zugelassenes Mikroskop oder medizinisches Mikroskop bezeichnet werden.

Ein weiterer Aspekt der vorliegenden Offenbarung betrifft ein Computerprogramm, umfassend Befehle, die bei der Ausführung durch eine Steuereinheit wie obenstehend beschrieben, bevorzugt eines Operationsmikroskops wie obenstehend beschrieben, bewirken, dass das System oder Operationsmikroskop, wie obenstehend beschrieben, das erfindungsgemäße Verfahren, wie obenstehend beschrieben, ausführen. Das Computerprogram umfasst bevorzugt Befehle, die bei der Ausführung durch eine Steuereinheit, wie obenstehend beschrieben, bevorzugt eines Operationsmikroskops, bewirken, dass das System oder Operationsmikroskop wie obenstehend beschrieben, das erfindungsgemäße Verfahren gemäß einer der bevorzugten Durchführungsformen, wie obenstehend beschrieben, ausführen. Das erfindungsgemäße Computerprogramm ist dabei bevorzugt in einem flüchtigen Speicher, beispielsweise einem RAM-Element oder in einem nicht-flüchtigen Speichermedium, wie beispielsweise einer CD-ROM, einem Flash-Speicher oder dergleichen, abgelegt.

Weitere bevorzugte Ausgestaltungen der Erfindung ergeben sich aus den übrigen, in den Unteransprüchen genannten Merkmalen und den nachfolgend erläuterten Figuren. Die verschiedenen in dieser Anmeldung genannten Ausführungsformen der Erfindung sind, sofern im Einzelfall nicht anders ausgeführt, mit Vorteil miteinander kombinierbar.

### Beschreibung der Figuren

Die Erfindung wird nachfolgend in Ausführungsbeispielen anhand der zugehörigen Zeichnungen erläutert. Es zeigen:
- Figur 1: eine schematische Darstellung eines Systems gemäß einer ersten Ausführungsform;
- Figur 2: eine schematische Darstellung eines Systems gemäß einer zweiten Ausführungsform;
- Figur 3: eine schematische Darstellung einer Probe und eines Anzeigemittels gemäß einer dritten Ausführungsform;
- Figur 4: eine schematische Darstellung eines Anzeigemittels gemäß einer vierten Ausführungsform;
- Figur 5: eine schematische Darstellung eines Anzeigemittels gemäß einer fünften Ausführungsform;
- Figur 6: eine schematische Darstellung eines Anzeigemittels und eines medizintechnischen Instruments gemäß einer fünften Ausführungsform; und
- Figur 7: ein schematisches Ablaufdiagram eines Verfahrens gemäß einer Durchführungsform.

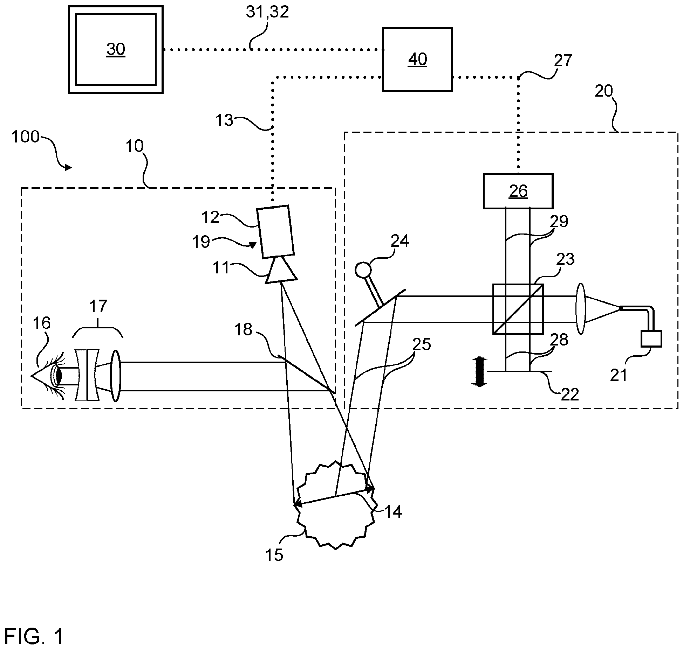

Figur 1 zeigt eine schematische Darstellung eines Systems 100 zur multimodalen Bilderfassung und -visualisierung gemäß einer ersten Ausführungsform.

Das System 100 weist ein operationsmikroskopisches System 10 mit einer Kamera 19, aufweisend eine Optik 11 und einen Bildsensor 12, auf. Die Kamera ist zum Erfassen eines zeitaufgelösten Bildsignals 13 eines ausgewählten Sichtfelds 14 einer Probe 15 ausgebildet. Bei der Kamera 19 handelt es sich beispielsweise um eine Hauptbeobachterkamera eines Operationsmikroskops, welches zusätzlich eine Umfeldkamera aufweisen kann. Ebenso kann das Operationsmikroskop einen zusätzlichen, mittels Strahlteiler 18 erzeugten Strahlengang aufweisen, der durch ein Okular 17 vom Auge eines Betrachters 16 beobachtet werden kann. Bei der Probe 15 handelt es sich insbesondere um einen Operationsbereich eines Patienten.

Das System 100 weist ferner ein OCT-System 20 mit einer breitbandigen Lichtquelle 21, beispielsweise einer Superlumineszenzdiode, auf. Das Licht der Lichtquelle 21 wird in ein Interferometer, aufweisend einen beweglichen Spiegel 22 und einen Strahlteiler 23, geleitet. Im Interferometer wird das Licht in einen Probenstrahl 25 und in einen Referenzstrahl 28 geteilt. Der Probenstrahl 25 wird mittels eines Scanspiegels 24 über die Probe 15 gerastert, wobei zumindest ein Abschnitt des Sichtfelds 14 der Probe 15 abgerastert wird. Der Referenzstrahl 28 wird auf den beweglichen Spiegel 22 gelenkt und von diesem zurück auf den Strahlteiler 23 reflektiert. Der Probenstrahl 25 interagiert mit der Probe 15, insbesondere dem Volumen der Probe 15 und wird von diesem zu dem Scanspiegel 24 zurückgestreut und von diesem auf den Strahlteiler 23 gelenkt. Dort werden der zurückgestreute Probenstrahl 25 und der reflektierte Referenzstrahl 28 zur Überlagerung gebracht, wobei ein Gangunterschied zwischen den überlagerten Strahlen 25, 28 durch den beweglichen Spiegel 22 eingestellt wird. Das so erzeugte Interferenzmuster 29 wird mittels eines Detektors 26, beispielsweise eines CCD-Detektors oder eines CMOS-Detektors, erfasst.

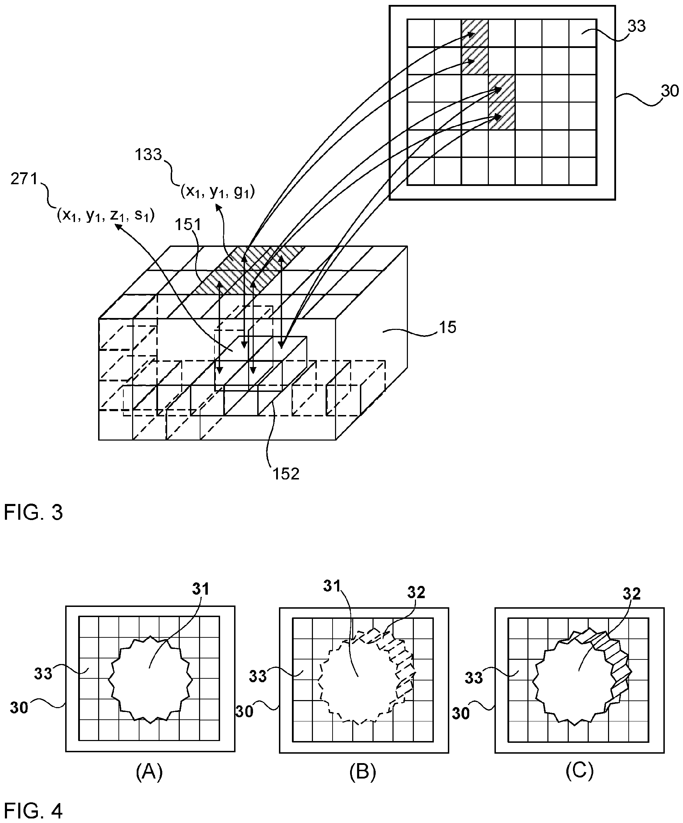

Das so erfasste zeitaufgelöste OCT-Signal 27 wird von dem Detektor 26 zur Steuereinheit 40 übermittelt, welches ebenfalls das zeitaufgelöste Bildsignal 13 von der Kamera 19 empfängt. Die Steuereinheit 40 ermittelt zu dem erfassten Bildsignal 13 korrespondierende Videobilddaten 31 und ein zu dem erfassten zeitaufgelösten OCT-Signal 27 korrespondierendes OCT-Bild 32, welches zumindest einem Abschnitt der Videobilddaten 31 entspricht. Die Steuereinheit 40 ist dazu eingerichtet, die Videobilddaten 31 auf dem zur zeitaufgelösten Anzeige von Bilddaten 31, 32 ausgebildeten Anzeigemittel 30 darzustellen. Die Steuereinheit ist auch dazu ausgebildet, das OCT-Bild 32 an der Position des zumindest korrespondierenden Abschnitts 60 der Videobilddaten 31 auf dem Anzeigemittel 30 darzustellen. Zur Erläuterung wird auf die Abbildung der Figur 3 verwiesen.

Figur 3 zeigt eine schematische Darstellung einer Probe 15 und eines Anzeigemittels 30 gemäß einer dritten Ausführungsform. Die Probe 15 weist dabei eine Vielzahl von Oberflächenelementen 151 und eine Vielzahl von Volumenelemente 152 auf, wobei bestimmte Volumenelemente 152 zu bestimmten Oberflächenelementen 151 korrespondieren. Eine beispielhafte Auswahl von Oberflächenelementen 151 ist schraffiert dargestellt und während die Mehrheit der Volumenelemente 152 mit gestrichenen Linien dargestellt sind, sind die vier zu den schraffierten Oberflächenelementen 151 korrespondierenden Volumenelemente 152 mit durchgezogenen Linien dargestellt. Ferner verbinden doppelseitige Pfeile diese Volumenelemente 152 mit den zugehörigen Oberflächenelementen 151.

Mit dem operationsmikroskopischen System 10 des Systems 100 ist insbesondere die Oberfläche der Probe 15 erfassbar, indem von dieser reflektiertes beziehungsweise rückgestreutes langwelliges (sichtbares) Licht über die Optik 11 auf den Bildsensor 12 der Kamera zurückgeworfen wird. Das mittels des Bildsensors 12 erfasste Bildsignal 13 weist eine Vielzahl von ersten Tupeln 133 auf, wobei sich eine Anzahl der ersten Tupeln 133 beispielsweise aus einer Auflösung des Bildsensors 12 ergibt. Jedes der ersten Tupeln 133 korrespondiert dabei zu einem der dargestellten Oberflächenelemente 151 und weist einen Wert einer Graustufe *gᵢ* auf, der eine Intensität des auf den Bildsensor 12 zurückgeworfenen Lichts entspricht. Anhand einer Kalibrierung beziehungsweise Registrierung des Bildsensors 12 relativ zu einem Koordinatensystem der Probe 15 (Patient) sind jedem der ersten Tupel 133 ferner zwei laterale Raumkoordinaten *xᵢ, yᵢ*, zugeordnet. In dem dargestellten Beispiel weist ein erstes Tupel 133 die lateralen Raumkoordinaten *x₁*, *y₁* und den Graustufenwert *g₁* auf.

Mit dem OCT-System 20 des Systems 100 ist insbesondere das Volumen der Probe 15 erfassbar, indem von dieser gestreutes kurzwelliges Licht des Probenstrahls 25 über den Scanspiegel 24 mittels des Interferometers mit dem Referenzstrahl 28 überlagert wird. Das so erzeugte und mittels dem Detektor 26 als zeitaufgelöstes OCT-Signal 27 erfasste Interferenzmuster weist eine Vielzahl von zweiten Tupeln 271 auf, wobei sich eine Anzahl der zweiten Tupeln 271 beispielsweise aus einer Anzahl der mit dem Scanspiegel 24 abgerasterten Punkte auf der Probe 15 ergibt. Jedes der zweiten Tupeln 271 korrespondiert dabei zu einem der dargestellten Volumenelemente 152 und weist einen Wert einer Streuintensität *sᵢ* auf. Anhand einer Kalibrierung beziehungsweise Registrierung des OCT-Systems 20 relativ zu einem Koordinatensystem der Probe 15 (Patient) sind jedem der zweiten Tupeln 271 ferner drei Raumkoordinaten *xᵢ, yᵢ, zᵢ* zugeordnet. In dem dargestellten Beispiel weist ein zweites Tupel 271 die Raumkoordinaten *x₁, y₁, z₁* und den Streuintensitätswert *s₁* auf.

Das in Figur 3 ferner dargestellte Anzeigemittel 30 weist eine Vielzahl von Pixeln 33 auf, insbesondere 42 Pixel mit 7 Pixeln in horizontaler Richtung und 6 Pixeln in vertikaler Richtung. In dem dargestellten Beispiel ergab sich aus der Auflösung des Bildsensors 12 eine Erfassung der Probenoberfläche durch das Bildsignal 13 in 21 Oberflächenelementen 151 mit 7 Oberflächenelementen 151 in horizontaler Richtung und 3 Oberflächenelementen 151 in vertikaler Richtung. Somit korrespondiert in den Videobilddaten 31 in horizontaler Richtung ein Pixel 33 zu einem Oberflächenelement 151 und korrespondieren in vertikaler Richtung zwei Pixel 33 zu einem Oberflächenelement 151. Zu den schraffiert dargestellten Oberflächenelementen 151 korrespondierende Pixel 33 sind ebenfalls schraffiert dargestellt und die Zuordnung ist ferner durch Pfeile verdeutlicht. Wie ferner durch Pfeile dargestellt, wird durch die Steuereinheit 40 des Systems 100 auch das zugehörige OCT-Bild 32 der zu den Oberflächenelementen 151 korrespondierenden Volumenelementen 152 so erzeugt und in den jeweiligen Pixeln 33 dargestellt, dass Videobilddaten 31 von bestimmten Oberflächenelementen 151 auf bestimmten Pixeln 33 dargestellt werden und OCT-Bilder 32 von zu den Oberflächenelementen 151 korrespondierenden Volumenelemente 152 ebenfalls auf den bestimmten Pixeln 33 dargestellt werden. Mithin werden korrespondierende Videobilddaten 31 und OCT-Bilder 32 am selben Ort des Anzeigemittels 30 dargestellt.

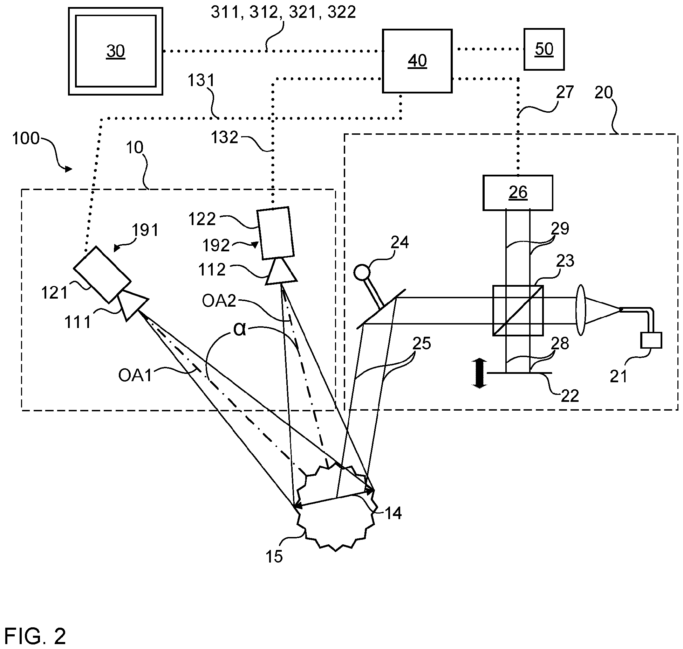

Figur 2 zeigt eine schematische Darstellung eines Systems 100 zur multimodalen Bilderfassung und -visualisierung gemäß einer zweiten Ausführungsform. Gleiche Komponenten sind mit gleichen Bezugszeichen wie in Figur 1 bezeichnet und auf eine wiederholte Beschreibung dieser Komponenten wird aus Gründen der Knappheit verzichtet.

Das System 100 der Figur 2 unterscheidet sich von dem der Figur 1 dadurch, dass das operationsmikroskopische System 10 eine erste Kamera 191 mit einer ersten Optik 111 und einem ersten Bildsensor 121 und eine zweite Kamera 192 mit einer zweiten Optik 112 und einem zweiten Bildsensor 122 aufweist. Mit jeder dieser Kameras 191, 192 ist ein Sichtfeld 14 der Probe 15 entlang einer optischen Achse erfassbar, so dass im Ergebnis ein stereoskopisches Bildsignal erfasst wird, welches ein erstes Bildsignal 131 und ein zweites Bildsignal 132 aufweist. Ein Stereowinkel α zwischen den optischen Achsen OA1, OA2 der beiden Kameras definiert dabei einen Tiefeneindruck beziehungsweise eine Tiefenwahrnehmung der anhand des stereoskopischen Bildsignals 131, 132 erstellten stereoskopischen Videobilddaten 31. Die stereoskopischen Videobilddaten 311, 312 weisen dabei zu dem ersten Bildsignal 131 korrespondierende erste Videobilddaten 311 und zu dem zweiten Bildsignal 132 korrespondierende zweite Videobilddaten 312 auf. Der Stereowinkel α hängt neben dem Abstand der Kameras zueinander von einem Arbeitsabstand zwischen den Kameras und der Probe 15 ab.

Ein zeitaufgelöstes OCT-Signal 27 wird in dem System 100 der Figur 2 in gleicher Weise erfasst, wie mit Bezug zu Figur 1 bereits beschrieben. Die Steuereinheit 40 ermittelt jedoch anhand des OCT-Signals 27 ein zu den ersten Videobilddaten 311 korrespondierendes erstes OCT-Bild 321 und ein zu den zweiten Videobilddaten 311 korrespondierendes zweites OCT-Bild 322. Das erste OCT-Bild 321 und das zweite OCT-Bild 322 werden dabei beispielsweise so erzeugt, als wären diese unter demselben Stereowinkel α erfasst worden, wie das erste und zweite Bildsignal 131, 132. Ebenfalls kann ein Stereowinkel der OCT-Bilder 321, 322 jedoch auch anhand einer mittels der Schnittstelle 50 erfassten Nutzereingabe abweichend eingestellt werden. Die ersten und zweiten Videobilddaten 311, 312 werden gemeinsam mit den ersten und zweiten OCT-Bildern 321, 322 gleichzeitig oder sequentiell auf dem Anzeigemittel 30 dargestellt, wobei ein Wechsel zwischen den OCT-Bildern 321, 322 und den Videobilddaten 311, 312 beispielsweise anhand einer Eingabe mittels Schnittstelle 50 erfolgt.

Eine schematische Darstellung eines Anzeigemittels 30 gemäß einer vierten Ausführungsform ist in Figur 4 dargestellt. Dabei zeigt Figur 4(A) die Darstellung von zur Probe 15 korrespondierenden Videobilddaten auf Pixeln 33 des Anzeigemittels 30, wie mit Bezug zu Figur 3 erläutert. Anhand einer Nutzereingabe, insbesondere mittels Schnittstelle 50, beispielsweise eines Fußschalters, erfasst die Steuereinheit 40, dass ein Wechsel von den Videobilddaten 31 zu dem OCT-Bild 32 der Probe 15 gewünscht ist. Die Steuereinheit 40 erhöht dabei sukzessive die Transparenz der Videobilddaten 31 und blendet gleichzeitig das korrespondierende OCT-Bild 32 mit abnehmender Transparenz an dem Ort der Videobilddaten 31 auf dem Anzeigemittel 30 ein. Eine Zwischenstufe mit einer gleichzeitigen und überlagerten Darstellung von halbtransparenten Videobilddaten 31 und halbtransparentem OCT-Bild 32 ist in Figur 4(B) dargestellt. In Figur 4(C) wird schließlich nur noch das OCT-Bild 32 mit einer Transparenz von 0%, also deckend, dargestellt.

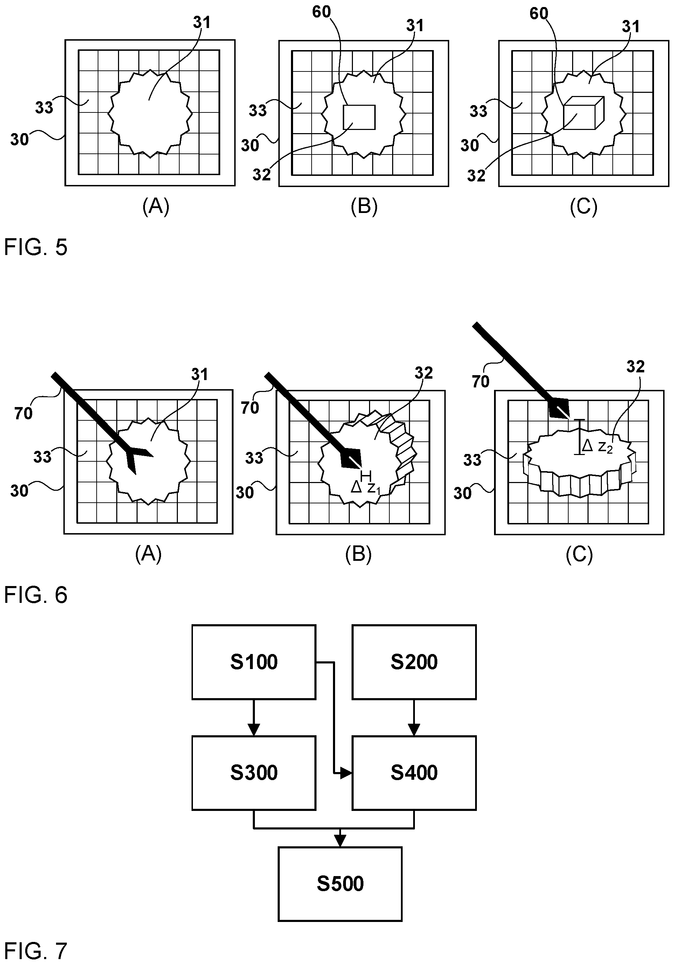

Eine schematische Darstellung eines Anzeigemittels 30 gemäß einer fünften Ausführungsform ist in Figur 5 dargestellt. Dabei zeigt Figur 5(A) die Darstellung von zur Probe 15 korrespondierenden Videobilddaten auf Pixeln 33 des Anzeigemittels 30 wie mit Bezug zu Figur 3 erläutert. Anhand einer Nutzereingabe, insbesondere mittels Schnittstelle 50, beispielsweise einem Sensor zum Erfassen von Kopf- und Augenbewegungen, erfasst die Steuereinheit 40, dass an einem bestimmten Ort der Darstellung ein Wechsel von den Videobilddaten 31 zu dem OCT-Bild 32 der Probe 15 gewünscht ist. Die Steuereinheit 40 ermittelt somit ein zu dem Abschnitt 60 der Videobilddaten 31 korrespondierendes OCT-Bild 32 und zeigt das OCT-Bild 32 an der Position des Abschnitts 60 auf dem Anzeigemittel 30 an. Dabei wird das OCT-Bild 32 zunächst in "*en*-face" Darstellung dargestellt, um einen nahtlosen Übergang zu dem in Draufsicht erfassten Videobilddaten 31 zu gewährleisten. Anhand einer weiteren Nutzereingabe mittels der Schnittstelle 50 erkennt die Steuereinheit 40 nachfolgend, dass die Darstellung des OCT-Bildes 32 des Abschnitts 60 in einer perspektivischen Darstellung gewünscht ist und wandelt daher eine Blickrichtung des OCT-Bilds 32 ab, so dass dieses, wie in Figur 4(C) dargestellt, als perspektivisches Volumenbild dargestellt wird. An der Stelle des OCT-Bilds 32 werden somit keine Videobilddaten 31 mehr angezeigt, insofern ist die Anzeige von OCT-Bild 32 und Videobilddaten 31 sequentiell. In dem das OCT-Bild 32 umgebenden Bereich des Anzeigemittels 30 werden jedoch weiterhin Videobilddaten 31 angezeigt, insofern erfolgt die Anzeige der Bilddaten 31, 32 gleichzeitig.

Eine schematische Darstellung eines Anzeigemittels 30 gemäß einer sechsten Ausführungsform ist in Figur 6 dargestellt. Dabei weist das System 100, wie in Figur 2 dargestellt, ferner ein medizintechnisches Instrument 70, insbesondere eine Pinzette 70, auf. In einer in Figur 6(A) dargestellten Annäherungsphase der Pinzette 70 an die Probe 15 erfolgt eine Darstellung der geöffneten Pinzette 70 und der Probe 15 auf dem Anzeigemittel 30 in Form von Videobilddaten 31 wie mit Bezug zu Figur 3 erläutert. Sobald durch die Steuereinheit 40 anhand von Sensorwerten und/oder anhand der Videobilddaten 31 erkannt wird, dass die Pinzette 70 geschlossen ist und ein vertikaler Abstand zwischen einer Spitze der Pinzette 70 und einer Oberfläche der Probe 15 gleich einem vorbestimmten Grenzwert Δz₁ oder geringer ist, wechselt die Steuereinheit 40 die Darstellung von Probe 15 und gegebenenfalls von Pinzette 70 auf dem Anzeigemittel 30 von den Videobilddaten 31 zu dem OCT-Bild 32, wie in Figur 6(B) dargestellt. In dem dargestellten Beispiel wird die Pinzette 70 genutzt, um einen Teil der Oberfläche der Probe 15 an- beziehungsweise abzuheben. Sobald durch die Steuereinheit 40 anhand von Sensorwerten und/oder anhand der Videobilddaten 31 erkannt wird, dass die Pinzette 70 geschlossen ist und ein vertikaler Abstand zwischen einer Spitze der Pinzette 70 und einer Oberfläche der Probe 15 außerhalb der an- beziehungsweise abgehobenen Oberfläche der Probe 15 (beispielsweise um einem Mindestabstand lateral beabstandet) gleich einem vorbestimmten Grenzwert Δz₂ oder größer ist, wechselt die Steuereinheit 40 zudem die Blickrichtung beziehungsweise Perspektive der Darstellung des OCT-Bildes 32, so dass dieses nun in einer seitlichen Perspektive als Volumenbild auf dem Anzeigemittel 30 anzeigt wird, wie es in Figur 6(C) dargestellt ist. Somit wird eine Interaktion von Operateur und Operationsmikroskop durch die vorteilhafte Darstellung der erfassten Signale, insbesondere des Bildsignals 13 und des OCT-Signals 27, kontinuierlich und objektiv verbessert, sei es durch die freie Wahl der örtlich registrierten dargestellten Bilddaten, also Videobilddaten 31 oder OCT-Bild 32, und/oder durch die Wahl von Art und Weise der Darstellung, beispielsweise in sequentieller, gleichzeitiger und/oder perspektivischer Weise.

Figur 7 zeigt ein schematisches Ablaufdiagram eines Verfahrens gemäß einer Durchführungsform. Das Verfahren weist einen ersten Verfahrensschritt S100 des Erfassens eines zeitaufgelösten Bildsignals 13 eines ausgewählten Sichtfelds 14 einer Probe 15 mittels eines operationsmikroskopischen Systems 10 auf. Dabei weist das Bildsignal 13 eine Vielzahl von ersten Tupeln 133 auf, die jeweils ein Oberflächenelement 151 der Probe 15 und zumindest einen zu dem Oberflächenelement 151 korrespondierenden Graustufenwert repräsentieren. In einem zweiten Schritt S200 des Verfahrens erfolgt das Erfassen eines zeitaufgelösten OCT-Signals 27 des ausgewählten Sichtfelds 14 mittels eines OCT Systems 20. Dabei weist das OCT-Signal 27 eine Vielzahl von zweiten Tupeln 271 auf, die jeweils ein Volumenelement 152 der Probe 15 und eine zu dem Volumenelement 152 korrespondierende Streuintensität repräsentieren. In einem Schritt S300 werden Videobilddaten 31 anhand der ersten Tupeln 133 und anhand einer Auflösung eines Anzeigemittels 30 mit einer Vielzahl von Pixeln 33 ermittelt, wobei in den Videobilddaten 31 bestimmte Pixel 133 bestimmten Oberflächenelementen 151 zugeordnet werden. In einem Schritt S400 wird ein zeitaufgelöstes OCT-Bild 32 anhand der zweiten Tupeln 271 sowie anhand der Auflösung des Anzeigemittels 30 und/oder anhand der Videobilddaten 31 ermittelt. Dies ist in Figur 7 durch die zwei zu Schritt S400 weisenden Pfeile dargestellt, wobei jedoch nur einer der Pfeile realisiert sein muss. Dabei werden zu den bestimmten Oberflächenelementen 151 korrespondierende Volumenelemente 152 den bestimmten Pixeln 33 zugeordnet, so dass das OCT-Bild 32 zumindest zu einem Abschnitt 60 der Videobilddaten 31 korrespondiert. In Schritt S500 erfolgt schließlich das Darstellen der Videobilddaten 31 auf dem Anzeigemittel 30 und das Darstellen des OCT-Bilds 32 an der Position des Abschnitts 60 auf dem Anzeigemittel 30.

### Bezugszeichenliste

- 10: operationsmikroskopisches System
- 11: Optik
- 111: erste Optik
- 112: zweite Optik
- 12: Bildsensor
- 121: erster Bildsensor
- 122: zweiter Bildsensor
- 13: zeitaufgelöstes Bildsignal
- 131: erstes Bildsignal
- 132: zweites Bildsignal
- 133: erste Tupel
- 14: Sichtfeld
- 15: Probe
- 151: Oberflächenelement
- 152: Volumenelement
- 16: Auge eines Betrachters
- 17: Okular
- 18: Strahlteiler
- 19: Kamera
- 191: erste Kamera
- 192: zweite Kamera
- OA1: erste optische Achse
- OA2: zweite optische Achse
- 20: OCT-System
- 21: breitbandige Lichtquelle
- 22: beweglicher Spiegel (Interferometer)
- 23: Strahlteiler (Interferometer)
- 24: Scanmechanismus (Scanspiegel)
- 25: Probenstrahl
- 26: Detektor
- 27: zeitaufgelöstes OCT-Signal
- 271: zweite Tupel
- 28: Referenzstrahl
- 29: Interferenzsignal
- 30: Anzeigemittel
- 31: Videobilddaten
- 311: erste Videobilddaten
- 312: zweite Videobilddaten
- 32: OCT-Bild
- 321: erstes OCT-Bild
- 322: zweites OCT-Bild
- 33: Pixel
- 40: Steuereinheit
- 50: Nutzerschnittstelle
- 60: Abschnitt
- 70: medizintechnisches Instrument

## Patentansprüche

1. Verfahren zur multimodalen Bilderfassung und -visualisierung, aufweisend die Verfahrensschritte:
Erfassen (S100) eines zeitaufgelösten Bildsignals (13) eines ausgewählten Sichtfelds (14) einer Probe (15) mittels eines operationsmikroskopischen Systems (10), wobei das Bildsignal (13) eine Vielzahl von ersten Tupeln (133) aufweist, die jeweils ein Oberflächenelement (151) der Probe (15) und zumindest einen zu dem Oberflächenelement (151) korrespondierenden Graustufenwert repräsentieren;
Erfassen (S200) eines zeitaufgelösten OCT-Signals (27) des ausgewählten Sichtfelds (14) mittels eines OCT-Systems (20), wobei das OCT-Signal (27) eine Vielzahl von zweiten Tupeln (271) aufweist, die jeweils ein Volumenelement (152) der Probe (15) und eine zu dem Volumenelement (152) korrespondierende Streuintensität repräsentieren;
Ermitteln (S300) von Videobilddaten (31) anhand der ersten Tupeln (133) und einer Auflösung eines Anzeigemittels (30) mit einer Vielzahl von Pixeln (33), wobei in den Videobilddaten (31) bestimmte Pixel (133) bestimmten Oberflächenelementen (151) zugeordnet werden;
Ermitteln (S400) eines zeitaufgelösten OCT-Bilds (32) anhand der zweiten Tupeln (271) und der Auflösung des Anzeigemittels (30) und/oder der Videobilddaten (31), wobei zu den bestimmten Oberflächenelementen (151) korrespondierende Volumenelemente (152) den bestimmten Pixeln (33) zugeordnet werden, so dass das OCT-Bild (32) zumindest zu einem Abschnitt (60) der Videobilddaten (31) korrespondiert;
Darstellen (S500) der Videobilddaten (31) auf dem Anzeigemittel (30) und Darstellen des OCT-Bilds (32) an der Position des Abschnitts (60) auf dem Anzeigemittel (30).

2. Verfahren nach Anspruch 1, ferner aufweisend die Verfahrensschritte:
Erfassen eines zeitaufgelösten ersten Bildsignals (131) des Sichtfelds (14) und eines zeitaufgelösten zweiten Bildsignals (132) des Sichtfelds (14);
Ermitteln zu dem ersten Bildsignal (131) korrespondierender erster Videobilddaten (311) und zu dem zweiten Bildsignal (132) korrespondierender zweiter Videobilddaten (312);
Ermitteln eines zeitaufgelösten ersten OCT-Bilds (321), das zumindest zu einem Abschnitt (60) der ersten Videobilddaten (311) korrespondiert und eines zeitaufgelösten zweiten OCT-Bilds (322), das zumindest zu dem Abschnitt (60) der zweiten Videobilddaten (312) korrespondiert;
stereoskopisches Darstellen der ersten Videobilddaten (311), der zweiten Videobilddaten (312), des zeitaufgelösten ersten OCT-Bilds (321) und des zeitaufgelösten zweiten OCT-Bilds (322) auf dem Anzeigemittel (30) mit gleicher Vergrößerung, gleicher Perspektive und/oder gleichem Stereowinkel α.

3. Verfahren nach Anspruch 2, wobei das operationsmikroskopische System (10) eine Stereokamera (11, 12) mit einem ersten Bildsensor (121) zum Erfassen des zeitaufgelösten ersten Bildsignals (131) des Sichtfelds (14) und einem zweiten Bildsensor (122) zum Erfassen des zeitaufgelösten zweiten Bildsignals (132) des Sichtfelds (14) aufweist und das Anzeigemittel (30) zum Darstellen stereoskopischer Bilddaten (31, 32) ausgebildet ist.

4. Verfahren nach Anspruch 2 oder 3, wobei
das erste und das zweite OCT-Bild (321, 322) an der Position des Abschnitts (60) auf dem Anzeigemittel (30) stereoskopisch dargestellt werden.

5. Verfahren nach einem der vorangehenden Ansprüche, wobei die Bildsignale (13) und OCT-Signale (27) anhand von Erfassungsparametern des OCT-Systems (20) und des operationsmikroskopischen Systems (10) örtlich registriert werden, und/oder Videobilddaten (31) und OCT-Bilder (32) mittels Bildanalyse örtlich registriert werden.

6. Verfahren nach einem der vorangehenden Ansprüche, wobei die Videobilddaten (31) und das zeitaufgelöste OCT-Bild (32) gleichzeitig auf dem Anzeigemittel (30) dargestellt werden.

7. Verfahren nach Anspruch 6, wobei die Videobilddaten (31) mit einer ersten Transparenzstufe und das zeitaufgelöste OCT-Bild (32) mit einer zweiten Transparenzstufe dargestellt werden.

8. Verfahren nach einem der vorangehenden Ansprüche, wobei die Videobilddaten (31) und das zeitaufgelöste OCT-Bild (32) sequentiell auf dem Anzeigemittel (30) dargestellt werden.

9. Verfahren nach einem der vorangehenden Ansprüche, wobei das zeitaufgelöste OCT-Bild (32) zu einem Abschnitt (60) der dargestellten Videobilddaten (31) korrespondiert und das OCT-Bild (32) in dem Abschnitt (60) und die Videobilddaten (31) auf dem Rest des Anzeigemittels (30) dargestellt werden.

10. Verfahren nach einem der vorangehenden Ansprüche, wobei ein Wechsel der Darstellung von Videobilddaten (31) und von zeitaufgelöstem OCT-Bild (32) anhand einer mittels einer Schnittstelle (50) zum Erfassen einer Nutzereingabe erfassten Nutzereingabe initiiert wird.

11. System (100) zur multimodalen Bilderfassung und -visualisierung, aufweisend:
ein operationsmikroskopisches System (10) mit einer Optik (11) und einem Bildsensor (12), ausgebildet zum Erfassen eines zeitaufgelösten Bildsignals (13) eines ausgewählten Sichtfelds (14) einer Probe (15), wobei das Bildsignal (13) eine Vielzahl von ersten Tupeln (133) aufweist, die jeweils ein Oberflächenelement (151) der Probe (15) und zumindest einen Graustufenwert repräsentieren;
ein zum Erfassen eines zeitaufgelösten OCT-Signals (27) des ausgewählten Sichtfelds (14) ausgebildetes OCT-System (20), wobei das OCT-Signal (27) eine Vielzahl von zweiten Tupeln (271) aufweist, die jeweils ein Volumenelement (152) der Probe (15) und eine Streuintensität repräsentieren;
ein zur zeitaufgelösten Anzeige von Bilddaten (31, 32) ausgebildetes Anzeigemittel (30) mit einer Vielzahl von Pixeln (33); und
eine Steuereinheit (40), die dazu eingerichtet ist:
zu dem erfassten Bildsignal (13) korrespondierende Videobilddaten (31) zu ermitteln und auf dem Anzeigemittel (30) darzustellen, wobei die Videobilddaten (31) anhand der ersten Tupeln (133) und einer Auflösung des Anzeigemittels (30) so ermittelt werden, dass bestimmte Pixel (33) bestimmte Oberflächenelemente (151) anzeigen,
anhand des erfassten OCT-Signals (27) ein zeitaufgelöstes OCT-Bild (32) zu ermitteln, das zumindest zu einem Abschnitt (60) der dargestellten Videobilddaten (31) korrespondiert und das OCT-Bild (32) an der Position des Abschnitts (60) auf dem Anzeigemittel (30) darzustellen, wobei das OCT-Bilder (32) anhand der zweiten Tupeln (271) und der Auflösung des Anzeigemittels (30) und/oder der Videobilddaten (31) so ermittelt wird, dass zu den bestimmten Oberflächenelementen (151) korrespondierende Volumenelemente (152) auf den bestimmten Pixeln (30) dargestellt werden.

12. System (100) nach Anspruch 11, ferner aufweisend ein medizintechnisches Instrument (70), wobei die Steuereinheit (40) dazu eingerichtet ist, eine Position, einen Typ und/oder einen Zustand des medizintechnischen Instruments (70) zu ermitteln und einen Wechsel der Darstellung von Videobilddaten (31) und von zeitaufgelöstem OCT-Bild (32) anhand der Position, des Typs und/oder des Zustands des medizintechnischen Instruments (70) zu initiieren.

13. System (100) nach Anspruch 11 oder 12, wobei die Steuereinheit (40) dazu eingerichtet ist, eine Zoomstufe des operationsmikroskopischen Systems (10) zu ermitteln und/oder eine Phase einer durchgeführten Operation zu ermitteln und einen Wechsel der Darstellung von Videobilddaten (31) und von zeitaufgelöstem OCT-Bild (32) anhand der Zoomstufe und/oder der Phase zu initiieren.

14. Computerprogramm, umfassend Befehle, die bei der Ausführung durch eine Steuereinheit eines Systems gemäß einem der Ansprüche 11 bis 13 bewirken, dass das System gemäß einem der Ansprüche 11 bis 13 ein Verfahren gemäß der Ansprüche 1 bis 10 ausführt.
